# FASCICULE DE BREVET EUROPEEN

(11) **EP 0 931 789 B1**
(45) Date de publication et mention de la délivrance du brevet: **02.05.2001**
(21) Numéro de dépôt: 99400062.8
(22) Date de dépôt: 12.01.1999
(51) Int. Cl.: C07C 319/24, C07C 321/14

(54) **Synthèse de disulfures et polysulfures organiques**
Herstellung von organischen Disulfiden und Polysulfiden
Preparation of organic disulfides and polysulfides

(30) Priorité: 22.01.1998 FR 9800660
(43) Date de publication de la demande: 28.07.1999
(73) Titulaire: Atofina, 92800 Puteaux (FR)
(72) Inventeur: Fremy, Georges, 64150 Os-Marsillon (FR)
(74) Mandataire: Leboulenger, Jean

(56) Documents cités:
- WO-A-97/21649
- WO-A-97/21673
- DATABASE CHEMABS CHEMICAL ABSTRACTS SERVICE, COLUMBUS, OHIO, US STN, accession no. 88:153338, XP002083536 & A.V. PASHKOV ET AL: RASSHIR. TEZISY DOKL. - VSES. SIMP. TERMODIN. IONNOGO OBMENA, 2ND,1975, pages 40-41,

## Description

La présente invention concerne le domaine des disulfures et polysulfures organiques R-Sₙ-R (avec n ≥ 2) et a plus particulièrement pour objet leur production par réaction de mercaptans avec le soufre en présence de résines basiques agissant comme catalyseurs selon la réaction :

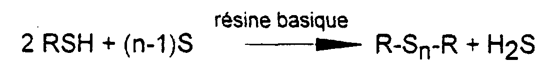

En présence de ces mêmes résines basiques, les disulfures et les polysulfures organiques de faible rang en soufre peuvent être transformés en polysulfures de rang supérieur en soufre par réaction avec le soufre. De même, en présence de ces mêmes résines basiques, les polysulfures organiques de rang élevé en soufre peuvent être transformés en polysulfures de rang inférieur en soufre par réaction avec des mercaptans.

La demande de brevet EP-A-337837 enseigne la préparation de disulfures et de polysulfures organiques catalysée par des résines organiques échangeuses d'anions comportant des groupes fonctionnels amine tertiaire ou ammonium quaternaire (actives sous forme hydroxyde). De telles résines, généralement sous la forme de grains ou de billes insolubles dans les milieux réactionnels liquides et donc facilement séparables en fin de réaction, permettent d'obtenir des disulfures organiques et des polysulfures par réaction du soufre élémentaire avec des mercaptans et également d'obtenir des polysulfures organiques à rang élevé en soufre par réaction du soufre élémentaire sur des polysulfures organiques de rang inférieur en soufre.

Selon la demande de brevet FR 2 742 144, l'utilisation de résines basiques à fonction amine primaire permet, comparativement aux résines à fonction amine tertiaire, d'obtenir un meilleur taux de conversion des réactifs et/ou une vitesse de réaction plus grande.

De même, la demande de brevet FR 2 742 145 préconise l'utilisation de résines fortement basiques à fonction guanidine ou amidine qui permet également, comparativement aux résines à fonction amine tertiaire, d'obtenir un meilleur taux de conversion des réactifs et/ou une vitesse de réaction plus grande.

Le but de la présente invention est d'améliorer encore ces résultats pour obtenir un meilleur taux de conversion des réactifs et/ou une vitesse de réaction plus grande.

Ce but est atteint par la mise en oeuvre de résines fonctionnalisées par des groupes éthylènediamine ou polyéthylènepolyamine.

Plus précisément, la présente invention propose un procédé de préparation des disulfures et polysulfures organiques par réaction du soufre sur un mercaptan ou sur un polysulfure de rang inférieur en soufre pour le transformer en polysulfure de rang supérieur, ou encore par réaction d'un mercaptan sur un polysulfure organique de rang élevé en soufre pour le transformer en polysulfure de rang inférieur en soufre, en présence d'un catalyseur sous forme de résine à fonctions basiques, caractérisé en ce que la résine est à base de polystyrène-divinylbenzène (PS-DVB) fonctionnalisée par des groupes éthylénediamine ou polyéthylénepolyamine, cette résine pouvant être représentée par la formule générale (I) : 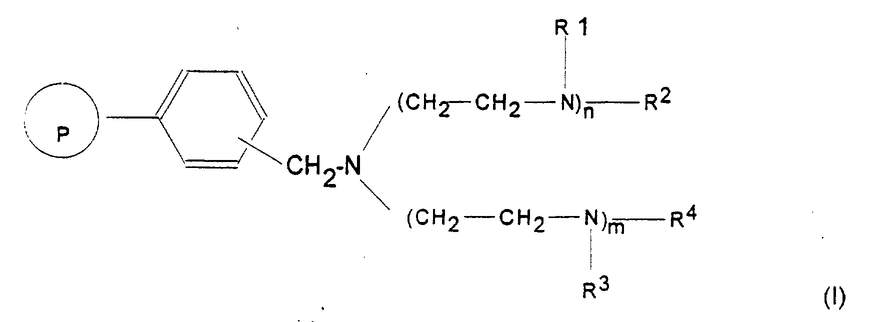 dans laquelle : 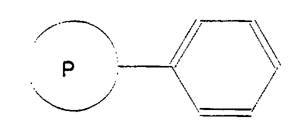 représente le support résine PS-DBV,
les symboles R¹, R², R³ et R⁴, identiques ou différents, représentent chacun un atome d'hydrogène ou un radical alkyle, cycloalkyle, aryle ou arylalkyle,
n est un nombre entier allant de 1 à 6,
m n'excède pas n et est égal à 0 ou est un nombre entier pouvant aller jusqu'à 5.

Les résines qui servent de matériaux de base pour la préparation des résines à fonction éthylènediamine ou polyéthylènepolyamine de formule générale (I) peuvent être des copolymères de PS-DVB ou des copolymères PS-DVB chlorométhylés qui, par des réactions chimiques appropriées décrites ci-après, sont transformés en résines à fonction éthylènediamine ou polyéthylènepolyamine selon l'invention.

Avec un faible taux de divinylbenzène (0,5 à 7 % en poids) comme agent de réticulation, on obtient des copolymères du type gel, tandis qu'avec des teneurs plus élevées en DVB, on peut obtenir des résines macroréticulées et de structure macroporeuse. Le taux de DVB peut être de 0,5 % à 60 % en poids par rapport au poids total du copolymère PS-DVB.

De préférence, les matériaux de base et, par voie de conséquence, les résines de formule générale (I) sont macroréticulées et de structure macroporeuse, car ces caractéristiques entraînent une meilleure activité catalytique que les résines de type gel.

Ces résines PS-DVB peuvent être chlorométhylées par l'éther chlorométhylique, selon des techniques connues et décrites dans la littérature, à des teneurs variable en chlore allant généralement de 1 à 20 % en poids de chlore par rapport au poids de résine chlorométhylée.

Les résines selon l'invention peuvent être préparées, suivant des techniques d'amination connues décrites dans la littérature, par réaction d'une résine PS-DVB chlorométhylée avec l'éthylènediamine ou une polyéthylènepolyamine de formule générale (II) : 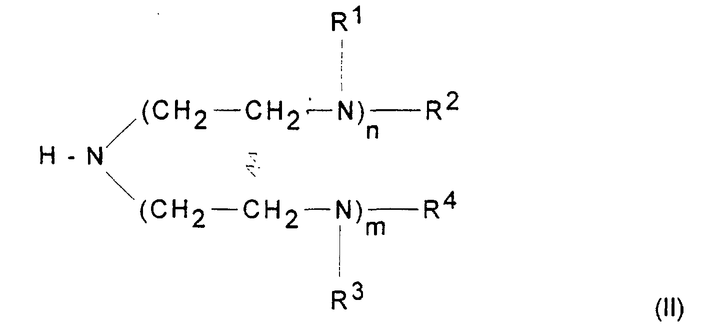 dans laquelle les symboles m, n, R¹, R², R³ et R⁴ ont les mêmes significations que précédemment. D'une manière générale, l'amine (ll) en excès est mise en solution dans un solvant de gonflement de la résine chlorométhylée (par exemple le tétrahydrofuranne) ; la résine cholorométhylée est introduite dans cette solution et le mélange réactionnel est agité pendant une durée allant de quelques heures à plusieurs jours (généralement de 4 heures à 4 jours) à une température qui peut aller d'environ 20°C au point d'ébullition du solvant, mais ne doit pas excéder la température limite de stabilité de la résine (généralement 100°C). En fin de réaction, la résine est lavée à la soude, puis avec un solvant de faible point d'ébullition (par exemple l'acétone) pour faciliter le séchage.

De préférence, on utilise une amine de formule (ll) dans laquelle n est un entier allant de 1 à 5, m est égal à 0, et les symboles R¹, R² et R⁴ sont des atomes d'hydrogène.

Les radicaux hydrocarbonés R des mercaptans, disulfures et polysulfures organiques sont généralement des groupes alkyle, cycloalkyle, aryle, arylalkyle ou alkylaryle. Ces radicaux R peuvent porter un ou plusieurs groupes fonctionnels tels que, par exemple, les atomes d'halogène et les groupes -OH, -OR', -SR', NR'R", -CN, -CHO, -COR' et -COOR', les symboles R' et R" désignant des radicaux aliphatiques de C₁ à C₁₂, cycloaliphatiques, aromatiques ou alkylaromatiques.

La présente invention s'applique notamment à la production de disulfures et polysulfures de dialkyle contenant au total de 2 à 40 atomes de carbone tels que, par exemple, les disulfures et les polysulfures de diméthyle, diéthyle, dipropyle, dibutyle, dipentyle, dihexyle, diheptyle, dioctyle, didécyle et didodécyle. Elle s'applique également à la préparation de disulfures et polysulfures cycloalkyliques tels que, par exemple, les disulfures et polysulfures de dicyclohexyle, ainsi qu'à la préparation de disulfures ou polysulfures aromatiques comme les disulfure et polysulfures de diphényle.

L'activité catalytique des résines mises en oeuvre dans la présente invention se manifeste à des teneurs très faibles de résine. Avantageusement, la résine est présente en une quantité allant de 0,01 à 20 parties en poids pour 100 parties en poids de mélange réactionnel, résine comprise.

Le procédé selon l'invention met en oeuvre une réaction qui peut être effectuée à une température de -10°C à 150°C. De préférence, la température est de +10°C à 120°C.

Les réactions peuvent être conduites à pression atmosphérique ou à des pressions supérieures pouvant atteindre 50 bars. Dans le cas de réactifs peu volatils et à faible tension de vapeur, la réaction peut être réalisée à des pressions inférieures à la pression atmosphérique, avec éventuellement la présence d'un gaz inerte tel que l'azote.

Le rapport molaire mercaptan/soufre dépend de la nature du mercaptan utilisé et du produit à préparer (disulfure ou polysulfure). Avantageusement ce rapport est de 0,3 à 10, de préférence de 0,4 à 6.

Quand on part d'un polysulfure organique de rang élevé en soufre pour le transformer en polysulfure organique de faible rang en soufre, par exemple en trisulfure R-S₃-R ou disulfure R-S₂-R par action du mercaptan correspondant, on utilise avantageusement un rapport molaire mercaptan/polysulfure allant de 2 à 10.

La production de disulfures ou de polysulfures organiques en présence de résines PS-DVB à fonctions éthylènediamine ou polyéthylènepolyamine peut être mise en oeuvre dans un réacteur agité ou tubulaire, suivant un procédé discontinu, soit par chargement des réactifs avant leur mise en réaction, soit par addition progressive de l'un des réactifs dans le réacteur, ou encore suivant un procédé continu avec addition contrôlée des réactifs.

Dans le cas où le soufre est l'un des réactifs (l'autre étant un mercaptan ou un polysulfure de faible rang en soufre), il peut être introduit sous la forme liquide ou solide.

En plus de la description qui précède, la présente invention sera mieux comprise à l'aide de la partie expérimentale qui suit à titre purement illustratif.

### EXEMPLE 1 : Préparation des résines selon l'invention

On a utilisé une résine macroporeuse PS-DVB chlorométhylée présentant les caractéristiques suivantes :
- surface spécifique : 22,5 m²/g de résine sèche
- diamètre moyen des pores : 2 nm
- volume des pores : 0,69 ml/g
- mmoles de chlore/g de résine : 5,4 mmol/g de résine sèche

On a pesé 10 g de résine chlorométhylée sèche (soit 54 mmoles de chlore) et les a mis en contact sous atmosphère d'azote avec 6,48 g (108 mmoles) d'éthylènediamine dilués dans 130 ml de tétrahydrofuranne (THF) préalablement séché sur tamis moléculaire. Le mélange réactionnel ainsi obtenu a été agité mécaniquement pendant 48 heures à 60°C.

Après refroidissement à 20°C, la résine a été filtrée et lavée successivement au THF, puis avec 20 ml d'une solution aqueuse de soude à 10 %, ensuite à l'eau jusqu'à neutralité et enfin à l'acétone, avant d'être séchée sous vide à 60°C jusqu'à poids constant.

On a ainsi obtenu une résine PS-DVB à fonctions éthylènediamine (ci-après résine EDA) contenant 7,6 % en poids d'azote, soit 2,70 mmoles d'éthylènediamine par gramme de résine sèche.

En opérant comme précédemment, mais en remplaçant l'éthylènediamine par une quantité molaire équivalente de diéthylènetriamine (11,2 g), de triéthylènetétramine (15,77 g), de tétraéthylènepentamine (20,41 g) ou de pentaéthylènehexamine (25,06 g), on a obtenu d'autres résines selon l'invention, respectivement identifiées comme résines DETA, TETA, TEPA et PEXA. Leurs caractéristiques sont rassemblées dans le tableau suivant.

Figurent également dans ce tableau les caractéristiques de résines PS-DVB selon l'art antérieur, à savoir :
- une résine à fonctions tétraméthylguanidine (ci-après résine TMG) préparée comme ci-dessus, mais en remplaçant l'éthylènediamine par 12,5 g de tétraméthylguanidine ;
- une résine à fonctions triazabicyclodécène (ci-après résine TBD) préparée comme décrit aux pages 23 et 24 de la demande de brevet FR 2 742 145.

| **Résine** | **N %** | **mmol/g de résine sèche** |
|---|---|---|
| | | |
| EDA | 7,6 | 2,7 mmol d'EDA/g |
| DETA | 9,0 | 2,13 mmol de DETA/g |
| TETA | 10,6 | 1,9 mmol de TETA/g |
| TEPA | 11,8 | 1,68 mmol de TEPA/g |
| PEXA | 11,6 | 1,38 mmol de PEXA/g |
| | | |
| | | |
| TMG | 8,74 | 2,08 mmol de TMG/g |
| TBD | 11,29 | 2,69 mmol de TBD/g |

### EXEMPLE 2 : Synthèse du di(tert-dodécyl)trisulfure par réaction du tert-dodécylpercaptan avec le soufre en présence de résines basiques

On a effectué des essais de production du di(tert-dodécyl)trisulfure dans des conditions expérimentales identiques en utilisant comme catalyseurs les résines EDA, DETA, TETA, TEPA et PEXA selon l'invention.

Ont également été effectués des essais comparatifs en utilisant les résines de l'art antérieur, à savoir :
- la résine Amberlyst A21 de Rohm et Haas qui est une résine PS-DVB de type macroporeux à fonctions -CH₂N(CH₃)₂ présentant une surface spécifique de 39,8 m²/g et 4,4 mmoles de fonctions amine tertiaire/g de résine sèche ;
- la résine Purolite A109 qui est une résine PS-DVB à fonctions -CH₂NH₂, macroréticulée et de structure macroporeuse (4,3 mmol de NH₂/g de résine sèche) ;
- les résines TMG et TBD selon la demande de brevet FR 2 742 145.

Ces essais ont été effectués dans un appareillage comprenant un réacteur double enveloppe en verre de 250 ml équipé :
- d'un fritté avant la vanne de vidange,
- d'une arrivée d'azote par une canne plongeante terminée par un fritté (débit d'azote contrôlé par un débitmètre à bille),
- d'un réfrigérant à circulation d'eau relié à l'aspiration de la hotte par un compte-bulle contenant de l'huile,
- d'un bain thermostaté permettant la circulation d'huile dans la double enveloppe,
- d'un agitateur en verre terminé par une ancre en PTFE et muni d'un moteur d'agitation à tachymètre,
- d'une sonde thermométrique dans une gaine de verre.

Après avoir mis le réacteur sous atmosphère d'azote, on y a introduit 6 g ou 15 g de résine (soit un ratio cata/RSH = 4 % ou 10 % respectivement), puis 151,2 g (750 mmol) de tertiododécylmercaptan (TDM). Le réacteur a ensuite été chauffé à 90°C, puis on a ajouté 24 g (750 mmol) de soufre en une seule fois. L'agitation étant maintenue à 500tr/min, la dissolution du soufre s'est effectuée en environ 30 minutes.

Après avoir mis en route le bullage d'azote (débit = 12 l/h) pour toute la durée de la réaction, on a prélevé des échantillons au cours du temps (t = 0 au moment de l'introduction du soufre) pour déterminer la teneur en mercaptan résiduel (% massique) par argentimétrie et la teneur en soufre libre par HPLC ; la teneur en mercaptan résiduel permet de déterminer le taux de conversion du TDM.

En fin de réaction, le polysulfure est filtré à chaud (40°C) sur le fritté du réacteur et la résine est prête pour être réutilisée avec une nouvelle charge de TDM et de soufre.

Les résultats obtenus avec les différentes résines sont rassemblés dans le tableau suivant.

| **Essai n°** | **Résine** | **% catalyseur** | **Conversion du TDM après 4 heures de réaction (%)** |
|---|---|---|---|
| | | | |
| 1 | A-21 | 4 | 65 |
| 2 | " | 10 | 69 |
| 3 | A-109 | 4 | 68 |
| 4 | " | 10 | 73 |
| 5 | TMG | 4 | 74 |
| 6 | TBD | 4 | 73 |
| | | | |
| | | | |
| 7 | EDA | 4 | 80 |
| 8 | " | 10 | 88 |
| 9 | DETA | 4 | 82 |
| 10 | " | 10 | 89 |
| 11 | TETA | 4 | 86 |
| 12 | " | 10 | 90 |
| 13 | TEPA | 4 | 86 |
| 14 | PEXA | 4 | 86 |

L'examen de ces résultats montre que les résines à fonctions éthylènediamine ou polyéthylènepolyamines selon l'invention (essais 7 à 14) ont une activité supérieure à celles des résines à fonctions amine tertiaire (essais 1 et 2), amine primaire (essais 3 et 4) ou guanidine (essais 5 et 6).

### EXEMPLE 3 : Synthèse du di(tert-dodécyl)pentasulfure par réaction du tert-dodécylmercaptan avec le soufre en présence de résines basiques

Dans le même appareillage qu'à l'exemple 2, on a introduit 6 g de résine (soit un ratio cata/RSH = 4 %) dans le réacteur préalablement mis sous atmosphère d'azote, puis 151,2 g (750 mmol) de TDM. Après avoir porté le réacteur à 90°C, on y a introduit 48 g (1,5 mole) de soufre en trois fois espacées de 10 minutes. L'agitation étant maintenue à 500 tr/min, la dissolution totale du soufre s'est faite en 45 minutes environ.

Après avoir mis en route le bullage d'azote (débit = 6 l/h) pour toute la durée de la réaction, on a ensuite procédé comme à l'exemple 2. Les résultats des essais sont rassemblés dans le tableau suivant.

| **Essai n°** | **Résine** | **Conversion du TDM après :** | |
|---|---|---|---|
| | | 1 heure | 3 heures |
| | | | |
| 15 | A-21 | 86 % | 97 % |
| 16 | TMG | - | 91 % |
| 17 | A-109 | - | 95 % |
| 18 | DETA | 94 % | 99 % |

L'examen de ce tableau montre que la résine selon l'invention (essai 18) donne de meilleurs résultats que les résines de la technique antérieure (essais 15 à 17).

## Revendications

1. Procédé de préparation de disulfures et polysulfures organiques R-Sₙ-R (avec n ≥ 2) par réaction du soufre sur un mercaptan R-SH ou sur un polysulfure de rang inférieur en soufre pour le transformer en polysulfure de rang supérieur, ou encore par réaction d'un mercaptan sur un polysulfure organique de rang élevé en soufre pour le transformer en polysulfure de rang inférieur en soufre, en présence d'un catalyseur sous forme de résine à fonctions basiques, caractérisé en ce que la résine est à base de polystyrène-divinylbenzène (PS-DVB) fonctionnalisée par des groupes éthylènediamine ou polyéthylènepolyamine, cette résine pouvant être représentée par la formule générale (I) : 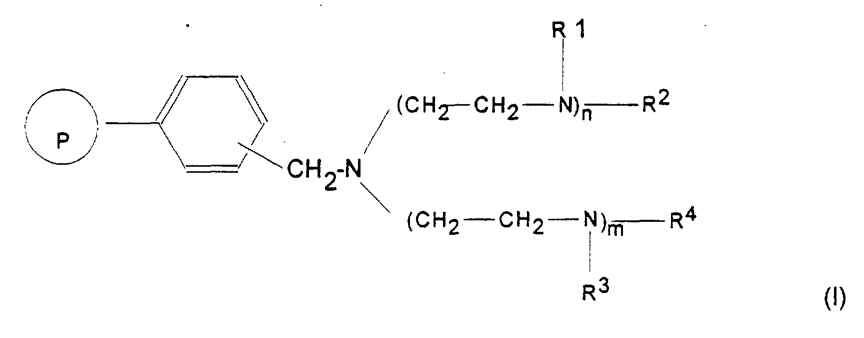 dans laquelle : 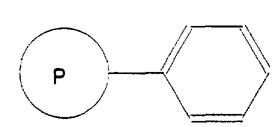 représente le support résine PS-DVB,
les symboles R¹, R², R³ et R⁴, identiques ou différents, représentent chacun un atome d'hydrogène ou un radical alkyle, cycloalkyle, aryle ou arylalkyle,
n est un nombre entier allant de 1 à 6,
m n'excède pas n et est égal à 0 ou est un nombre entier pouvant aller jusqu'à 5.

2. Procédé selon la revendication 1, caractérisé en ce que l'on utilise une résine macroréticulée et de structure macroporeuse.

3. Procédé selon la revendication 1 ou 2, caractérisé en ce que les radicaux hydrocarbonés R des mercaptans, disulfures et polysulfures organiques sont des groupes alkyle, cycloalkyle, aryle ou arylalkyle, portant éventuellement un ou plusieurs groupes fonctionnels.

4. Procédé selon l'une des revendications 1 à 3, caractérisé en ce que la résine est présente en une quantité allant de 0,01 à 20 parties en poids pour 100 parties en poids de mélange réactionnel, résine comprise.

5. Procédé selon l'une des revendications 1 à 4, caractérisé en ce que la réaction est effectuée à une température comprise entre -10°C et 150°C, de préférence entre +10°C et 120°C.

6. Procédé selon l'une des revendications 1 à 5 pour la préparation d'un disulfure ou polysulfure à partir d'un mercaptan et de soufre, caractérisé en ce que le rapport molaire mercaptan/soufre est compris entre 0,3 et 10, de préférence entre 0,4 et 6.

7. Procédé selon l'une des revendications 1 à 5 pour la préparation d'un polysulfure de faible rang en soufre à partir d'un polysulfure de rang élevé en soufre, caractérisé en ce que le rapport molaire mercaptan/polysulfure est compris entre 2 et 10.

8. Procédé selon l'une des revendications 1 à 7 dans lequel la résine fonctionnalisée résulte de la réaction d'une résine PS-DVB chlorométhylée avec un excès d'éthylènediamine ou d'une polyéthylènepolyamine de formule générale (II) : 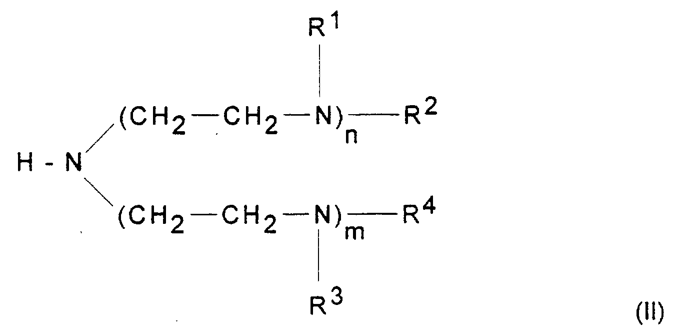 dans laquelle les symboles m, n, R¹, R², R³ et R⁴ ont les mêmes significations que dans la revendication 1.

9. Procédé selon la revendication 8 dans lequel n est un entier allant de 1 à 5, m est égal à 0, et les symboles R¹, R² et R⁴ sont des atomes d'hydrogène.

## Claims

1. Process for the preparation of organic disulphides and polysulphides R-Sₙ-R (with n ≥ 2) by reaction of sulphur with a mercaptan R-SH or with a polysulphide of lower sulphur order in order to convert it into polysulphide of higher order, or alternatively by reaction of a mercaptan with an organic polysulphide of high sulphur order in order to convert it into polysulphide of lower sulphur order, in the presence of a catalyst in the form of a resin with basic functions, characterized in that the resin is based on polystyrene-divinylbenzene (PS-DVB) functionalized with ethylenediamine or polyethylenepolyamine groups, it being possible for this resin to be represented by the general formula (I): 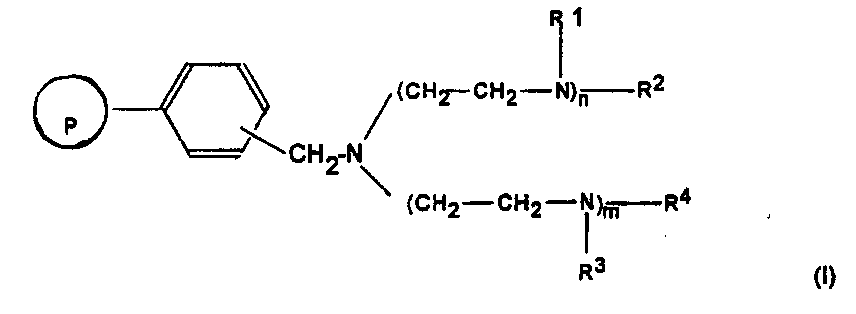 in which: 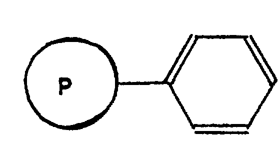 represents the PS-DVB resin support,
the symbols R¹, R², R³ and R⁴, which may be identical or different, each represent a hydrogen atom or an alkyl, cycloalkyl, aryl or arylalkyl radical,
n is an integer ranging from 1 to 6,
m does not exceed n and is equal to 0 or is an integer which can be up to 5.

2. Process according to Claim 1, characterized in that a macrocrosslinked resin of macroporous structure is used.

3. Process according to Claim 1 or 2, characterized in that the hydrocarbon radicals R of the organic mercaptans, disulphides and polysulphides are alkyl, cycloalkyl, aryl or arylalkyl groups optionally bearing one or more functional groups.

4. Process according to one of Claims 1 to 3, characterized in that the resin is present in an amount ranging from 0.01 to 20 parts by weight per 100 parts by weight of reaction mixture, including resin.

5. Process according to one of Claims 1 to 4, characterized in that the reaction is carried out at a temperature of between -10°C and 150°C, preferably between +10°C and 120°C.

6. Process according to one of Claims 1 to 5 for the preparation of a disulphide or polysulphide starting with a mercaptan and sulphur, characterized in that the mercaptan/sulphur molar ratio is between 0.3 and 10, preferably between 0.4 and 6.

7. Process according to one of Claims 1 to 5 for the preparation of a polysulphide of low sulphur order starting with a polysulphide of high sulphur order, characterized in that the mercaptan/ polysulphide molar ratio is between 2 and 10.

8. Process according to one of Claims 1 to 7, in which the functionalized resin results from the reaction of a chloromethylated PS-DVB resin with an excess of ethylenediamine or of a polyethylenepolyamine of general formula (II): 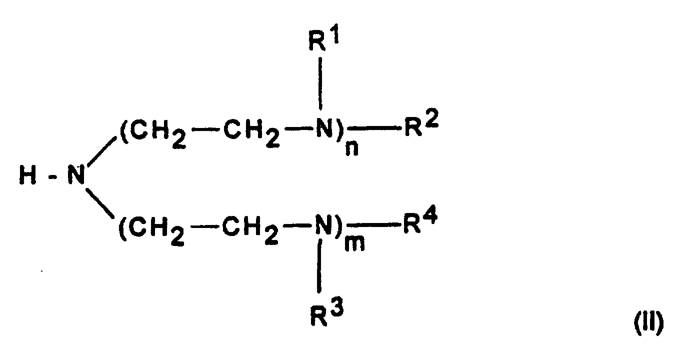 in which the symbols m, n, R¹, R², R³ and R⁴ have the same meanings as in Claim 1.

9. Process according to Claim 8, in which n is an integer ranging from 1 to 5, m is equal to 0 and the symbols R¹, R² and R⁴ are hydrogen atoms.

## Patentansprüche

1. Verfahren zur Herstellung von organischen Di- und Polysulfiden R-Sₙ-R (mit n ≥ 2) durch Einwirkung von Schwefel auf ein Mercaptan R-SH oder ein Polysulfid mit niedrigem Schwefelgehalt, um es in ein Polysulfid mit höherem Schwefelgehalt umzuwandeln, oder auch durch Einwirkung eines Mercaptans auf ein organisches Polysulfid mit hohem Schwefelgehalt, um es in ein Polysulfid mit niedrigem Schwefelgehalt umzuwandeln, in Gegenwart eines Katalysators in Form eines basischen Harzes, dadurch gekennzeichnet, daß das Harz auf der Grundlage von Polystyrol-Divinylbenzol (PS-DVB) durch Ethylendiamin- oder Polyethylenpolyamingruppen funktionell wirksam gemacht ist, wobei dieses Harz durch die allgemeine Formel (I) dargestellt werden kann: 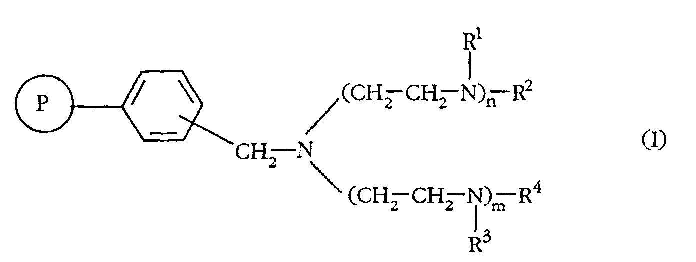 in der: 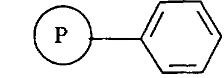 das Trägerharz PS-DVB bedeutet,
die gleichen oder verschiedenen Symbole R¹, R², R³ und R⁴ ein jedes ein Wasserstoffatom oder einen Alkyl-, Cycloalkyl-, Aryl- oder Arylalkylrest bedeuten,
n eine ganze Zahl von 1 bis 6 ist,
m nicht größer als n und gleich 0 oder eine ganze Zahl bis 5 ist.

2. Verfahren nach Anspruch 1, dadurch gekennzeichnet, daß man ein Harz verwendet, das makrovernetzt ist und eine makroporöse Struktur hat.

3. Verfahren nach Anspruch 1 oder 2, dadurch gekennzeichnet, daß die kohlenwasserstoffhaltigen Reste R der Mercaptane sowie der organischen Di- und Polysulfide Alkyl-, Cycloalkyl-, Aryl- oder Arylalkylgruppen gegebenenfalls mit einer oder mehreren funktionellen Gruppen sind.

4. Verfahren nach einem der Ansprüche 1 bis 3, dadurch gekennzeichnet, daß das Harz in einer Menge von 0,01 bis 20 Gew.-Teilen, bezogen auf 100 Gew.-Teile des Reaktionsgemisches einschließlich des Harzes, vorliegt.

5. Verfahren nach einem der Ansprüche 1 bis 4, dadurch gekennzeichnet, daß die Reaktion bei einer Temperatur zwischen -10 °C und 150 °C, vorzugsweise zwischen +10 °C und 120 °C, durchgeführt wird.

6. Verfahren nach einem der Ansprüche 1 bis 5 zur Herstellung eines Di- oder Polysulfids aus einem Mercaptan und Schwefel, dadurch gekennzeichnet, daß das Molverhältnis Mercaptan/Schwefel zwischen 0,3 und 10, vorzugsweise zwischen 0,4 und 6, liegt.

7. Verfahren nach einem der Ansprüche 1 bis 5 für die Herstellung eines Polysulfids mit niedrigem Schwefelgehalt aus einem Polysulfid mit hohem Schwefelgehalt, dadurch gekennzeichnet, daß das Molverhältnis Mercaptan/ Polysulfid zwischen 2 und 10 liegt.

8. Verfahren nach einem der Ansprüche 1 bis 7, in dem das funktionell wirksam gemachte Harz aus der Reaktion eines chlormethylierten PS-DVB-Harzes mit einem Überschuß an Ethylendiamin oder einem Polyethylenpolyamin der allgemeinen Formel (II) stammt: 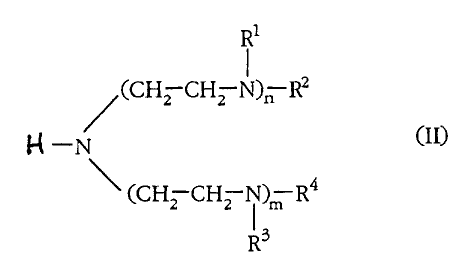 in der die Symbole m, n, R¹, R², R³ und R⁴ dieselben Bedeutungen wie in Anspruch 1 haben.

9. Verfahren nach Anspruch 8, in dem n eine ganze Zahl von 1 bis 5 und m gleich 0 ist und die Symbole R¹, R² und R⁴ Wasserstoffatome sind.
